# EUROPEAN PATENT APPLICATION

(11) **EP 2 868 274 A1**
(43) Date of publication of application: **06.05.2015**
(21) Application number: 14170290.2
(22) Date of filing: 28.05.2014
(51) Int. Cl.: A61B 5/18

(54) **Systems and methods for detecting pilot over focalization**

(30) Priority: 04.11.2013 US 201314070895
(71) Applicant: AIRBUS OPERATIONS (S.A.S), 31060 Toulouse (FR); Airbus (S.A.S.), 31700 Blagnac (FR)
(72) Inventor: Christophe, Laure, 31770 COLOMIERS (FR); Causse, Mickaël, 30000 Nimes (FR); Dehais, Frédéric, 31100 TOULOUSE (FR); Merle, Jean-Michel, 31320 VIEILLE-TOULOUSE (FR); Pellerin, Philippe, 31460 AURIAC SUR VENDINELLE (FR); Beaucamp, Justine, 31000 TOULOUSE (FR)
(74) Representative: Gicquel, Olivier Yves Gérard

(57) **Abstract**

Methods and systems are provided for determining over focalization of a pilot of an aircraft. The method includes receiving human parameter data (120,122,124,126) from one or more systems that observe conditions associated with the pilot and receiving controls data (128) that indicates an interaction of the pilot with one or more control systems of the aircraft. The method includes, based on the human parameter data (120,122,124,126) and the controls data (128), generating at least one alert. The method also includes communicating the at least one alert to the pilot to change a focus of the pilot.

## Description

The present disclosure generally relates to mobile platforms, such as aircraft, and more particularly relates to systems and methods for detecting pilot over focalization.

Many aircrafts are operated by one or more pilots that aid in ensuring the safe and smooth operation of the aircraft. The operation of the aircraft may be stressful, and the pilots may experience a high workload. In certain instances, a pilot may be overly focused on a particular task, and due to fatigue or other circumstances, the pilot may not be able to direct his/her attention to other tasks. This behavior is called perseveration syndrome and may lead to a reduction of performance in monitoring, tracking and auditory discrimination.

Accordingly, it is desirable to provide systems and methods to detect and correct pilot over focalization. Furthermore, other desirable features and characteristics of the present disclosure will become apparent from the subsequent detailed description and the appended claims, taken in conjunction with the accompanying drawings and the foregoing technical field and background.

A method is provided for determining over focalization of a pilot of an aircraft. The method includes receiving human parameter data from one or more systems that observe conditions associated with the pilot and receiving controls data that indicates an interaction of the pilot with one or more control systems of the aircraft. The method includes, based on the human parameter data and the controls data, generating at least one alert. The method also includes communicating the at least one alert to the pilot to change a focus of the pilot.

A system is provided for determining over focalization of a pilot. The system includes at least one system that observes a condition associated with the pilot and generates human parameter data based on the observed condition and a user input device for receiving a response from the pilot. The system also includes a control module that generates a first alert based on the human parameter data, and generates a second alert. The second alert is generated if no response is received to the first alert.

The exemplary embodiments will hereinafter be described in conjunction with the following drawing figures, wherein like numerals denote like elements, and wherein:
- Fig. 1 is a functional block diagram illustrating a mobile platform, such as an aircraft, that includes a system for detecting pilot focalization in accordance with various embodiments;
- Fig. 2 is a dataflow diagram illustrating a control system of the system for detecting pilot focalization in accordance with various embodiments; and
- Fig. 3 is a flowchart illustrating a control method of the system for detecting pilot focalization in accordance with various embodiments.

The following detailed description is merely exemplary in nature and is not intended to limit the application and uses. Furthermore, there is no intention to be bound by any expressed or implied theory presented in the preceding technical field, background, brief summary or the following detailed description. As used herein, the term module refers to any hardware, software, firmware, electronic control component, processing logic, and/or processor device, individually or in any combination, including without limitation: application specific integrated circuit (ASIC), an electronic circuit, a processor (shared, dedicated, or group) and memory that executes one or more software or firmware programs, a combinational logic circuit, and/or other suitable components that provide the described functionality.

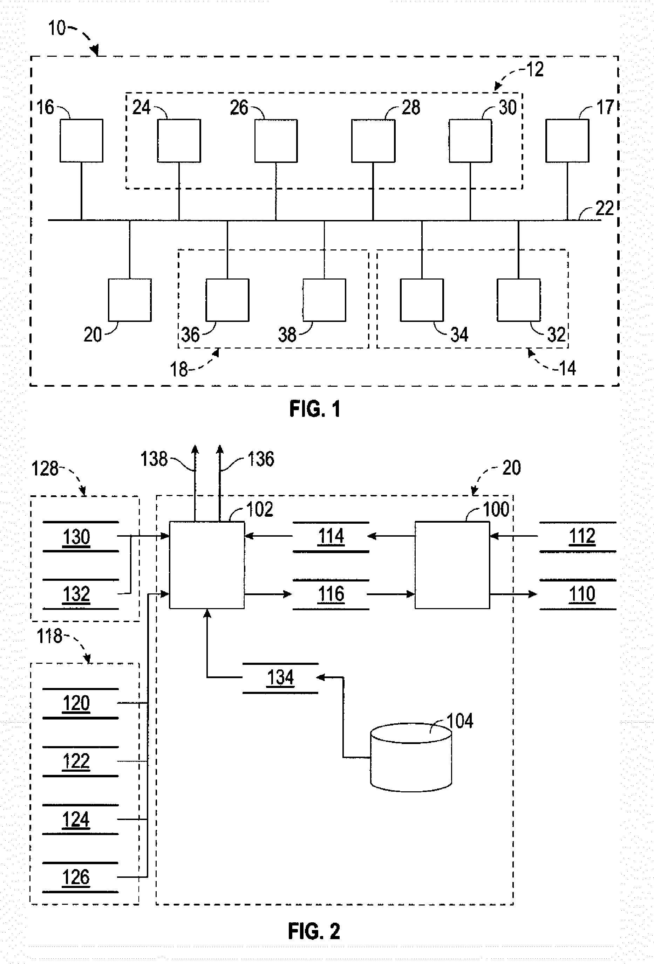

With reference to Fig. 1, a mobile platform, such as an aircraft 10, is shown. It should be noted that while an aircraft 10 is described and illustrated herein, the present teachings are applicable to any suitable mobile platform, including, but not limited to, a ship, train, bus, etc. In one example, the aircraft 10 includes a human monitoring system 12, a flight control system 14, an automatic flight system 16, a notification system 17 and a user interface 18 that provide input to and/or receive one or more control signals and/or data from a focalization control module 20. The human monitoring system 12, flight control system 14, automatic flight system 16, notification system 17 and user interface 18 are in communication with the focalization control module 20 over an interconnection architecture 22, or arrangement that facilitates transfer of data, commands, power, etc. As will be discussed in greater detail herein, the focalization control module 20 receives inputs from the human monitoring system 12, flight control system 14 and user interface 18, and based on these inputs generates one or more control signals for the automatic flight system 16 and the notification system 17, and generates alert data for the user interface 18. Generally, the focalization control module 20 enables the detection of an operator or pilot's focalization on a single task and provides outputs to change the focus of the pilot. In other words, the focalization control module 20 detects a pilot's perseverance syndrome behavior or amount of attention tunneling (i.e. amount of focus on a single item of a display or a single task) and provides output that causes the pilot to shift his/her focus to another item of a display or another task. It should be noted that while the system illustrated below and described herein generally refers to detecting the focalization of a single pilot, the system can be used to detect the focalization of more than one pilot. Thus, the following description is merely exemplary.

With continued reference to Fig. 1, the human monitoring system 12 provides the focalization control module 20 with data regarding the one or more pilots of the aircraft 10. In one example, the human monitoring system 12 includes a perspiration monitoring system 24, an eye monitoring system 26, a heart rate monitor or sensor 28 and a cerebral activity monitoring system 30. It should be noted that the systems and devices included in the human monitoring system 12 are merely exemplary, as the human monitoring system 12 may include any suitable system or device for observing conditions of the pilot and generating sensor signals and/or data based thereon for determining the pilot's focalization. In addition, it should be noted that multiple conditions of the pilot may be observed by a single system or device.

The perspiration monitoring system 24 comprises any suitable system, device or sensor capable of observing an amount of moisture present on a portion of the pilot, including, but not limited to, a face of the pilot. In one example, the perspiration monitoring system 24 comprises a sensor that observes moisture on the portion of the pilot and generates sensor signals based thereon. In this example, the sensor of the perspiration monitoring system 24 may be coupled to headwear worn by the pilot during the operation of the aircraft 10, coupled to a portion of the flight control system 14 (e.g. arranged on tactile pads associated with the flight control system 14), coupled to an earpiece worn by the pilot during the operation of the aircraft 10, etc. Alternatively, the perspiration monitoring system 24 may comprise an optical sensor, such as a camera, including, but not limited to, a complementary metal-oxide-semiconductor (CMOS) infrared camera, which observes the pilot and generates video data, which is analyzed by the focalization control module 20 or other modules associated with the aircraft 10 to determine an amount of moisture present on the portion of the pilot observed by the camera.

The eye monitoring system 26 comprises any suitable system, device or sensor that observes an eye condition for generating oculometrics data of the pilot, including, but not limited to, eye movement, focus, saccades, pupil diameter, blink rate, switching rate, etc. In one example, the eye monitoring system 26 comprises an optical sensor, such as a camera, including, but not limited to, a CMOS infrared camera. In this example, the camera of the eye monitoring system 26 observes conditions associated with the eyes of the pilot and generates video data based thereon. The video data is then analyzed by the focalization control module 20 and/or other modules associated with the aircraft 10 to determine one or more of the movement of the eyes, focus of the eyes, saccades of the eyes, a diameter of the pupils of the eyes, a blink rate of the eyes, a switching rate of the eyes, etc. It should be noted that the camera of the eye monitoring system 26 may be the same camera employed by the perspiration monitoring system 24, or that separate cameras may be used.

The heart rate sensor 28 comprises any suitable device that observes a heart rate of the pilot and generates sensor signals based thereon. For example, the heart rate sensor 28 comprises a band worn by the pilot, which includes a heart rate sensor as is generally known in the art. Alternatively, the heart rate sensor 28 may be integrated into one or more touch pads or contact pads associated with the flight control system 14, such that sensor signals are generated regarding the heart rate of the pilot through the pilot's continued contact with the contact pads.

The cerebral activity monitoring system 30 comprises any suitable system or device that observes a cerebral activity of a brain of the pilot and generates data or sensor signals based thereon. In one example, the cerebral activity monitoring system 30 comprises an electroencephalography (EEG) device. In this example, one or more sensors are coupled to the head of the pilot and sensor signals are generated based on the observed electrical activity in at least one of the theta band and alpha band. Alternatively, the cerebral activity monitoring system 30 comprises an EEG-Evoked Potentials (EP) device for observing electrical activity associated with the brain of the pilot and generating sensor signals based thereon. In other embodiments, the cerebral activity monitoring system 30 includes a simulated performance intensity function (SPIf) device or a functional optical brain imaging (fNIR) device.

With continued reference to Fig. 1, the flight control system 14 comprises various systems or devices that receive user input by the pilot for the proper operation and flight control of the aircraft 10. In one example, the flight control system 14 includes a flight control input system 32 and a switch system 34. Generally, the flight control input system 32 comprises the primary flight controls for the operation and flight of the aircraft 10. In one example, the flight control input system 32 includes, but is not limited to, a stick that receives pilot input to control the roll and pitch of the aircraft 10, a rudder control that receives pilot input to control yaw of the aircraft 10 through movement of a rudder associated with the aircraft 10, a throttle that receives pilot input to control engine speed or thrust of the aircraft 10, etc. It should be noted that the flight control input system 32 may also include secondary flight controls, including, but not limited to a wheel or other device to control elevator trim, a lever to control wing flaps, etc. As will be discussed herein, the pilot input received by the flight control input system 32 or pilot interaction with the flight control input system 32 is used by the focalization control module 20 to determine a focalization of the pilot and/or to adjust or change a focalization of the pilot.

The switch system 34 comprises one or more switches that receive input from the pilot during the operation of the aircraft 10. Generally, the one or more switches of the switch system 34 are mechanical, electrical or combinations thereof. In the example of an electrical switch, the input from the pilot or interaction of the pilot with the switch generates one or more signals that are transmitted to a switch control module, and the switch control module generates control signals to control one or more components of the aircraft 10 based on input from the pilot to the switch. For example, the one or more switches include, but are not limited to, a switch to activate one or more actuators (e.g. pumps) associated with the aircraft 10, one or more switches for interacting with an autopilot system or a flight director associated with the aircraft 10, etc. As will be discussed herein, the pilot input received by the switch system 34 can be used by the focalization control module 20 to determine a focalization of the pilot and/or to adjust or change a focalization of the pilot.

The automatic flight system 16 comprises one or more systems that automatically control the operation and flight of the aircraft 10. Thus, the automatic flight system 16 generally comprises an automatic flight control system (AFCS) for automatically controlling elevators, rudder, ailerons and other systems of the aircraft 10 as generally known to one skilled in the art. The automatic flight system 16 may also include one or more systems for automatically changing a flight plan of the aircraft 10 based on surrounding conditions along the current flight plan.

The notification system 17 is in communication with the focalization control module 20 over the interconnection architecture 22. The notification system 17 includes one or more systems or devices that communicate an alert to a pilot of the aircraft 10 through any suitable mechanism, such as haptic and/or visual. Thus, the notification system 17 is generally located in a cockpit of the aircraft 10. In one example, the notification system 17 includes a control module that receives one or more control signals from the focalization control module 20 and generates one or more control signals to activate a haptic device or visual device based on the received control signals. For example, the haptic device includes, but is not limited to, a vibration device coupled to a pilot's seat, and the visual device includes, but is not limited to, a lighting device in the cockpit of the aircraft 10. As will be discussed, the notification system 17 communicates one or more alerts to the pilot of the aircraft 10 based on the detection of focalization by the focalization control module 20.

The user interface 18 allows the pilot of the aircraft 10 to interface with various systems of the aircraft 10. Thus, the user interface 18 is generally located within a cockpit of the aircraft 10. The user interface 18 includes a user input device 36 and a display 38. The user input device 36 is any suitable device capable of receiving user input, for example, from the pilot of the aircraft 10. The user input device 36 includes, but not limited to, a keyboard, a microphone, a touchscreen layer associated with the display 38, or other suitable device to receive data and/or commands from the pilot. Of course, multiple user input devices 36 can also be utilized. The display 38 comprises any suitable technology for displaying information, including, but not limited to, a liquid crystal display (LCD), organic light emitting diode (OLED), plasma, or a cathode ray tube (CRT). As a nonlimiting example, the display 28 comprises a navigation display, a primary flight display or a horizontal display associated with the aircraft 10. As will be discussed in greater detail herein, the user interface 18 receives user input data from the user input device 36 and provides this user input to the focalization control module 20. The focalization control module 20 also outputs one or more alerts for the pilot over the interconnection architecture 22 for display on the display 38.

As will be discussed in detail with regard to Fig. 2, the focalization control module 20 receives data from the user input device 36, flight control input system 32 and switch system 34, along with data and/or sensor signals from the human monitoring system 12, such as the perspiration monitoring system 24, the eye monitoring system 26, heart rate sensor 28 and/or the cerebral activity monitoring system 30. Based on the data and sensor signals, the focalization control module 20 determines a focalization state of the pilot and outputs one or more alerts to the user interface 18 and/or one or more control signals to the notification system 17 to redirect or change the focus of the pilot. The focalization control module 20 also outputs one or more control signals to the automatic flight system 16 based on the pilot input in response to the alerts. Thus, the focalization control module 20 enables the detection of a pilot's focalization and provides methods to redirect or change the pilot's focus.

Referring now to Fig. 2 and with continued reference to Fig. 1, a dataflow diagram illustrates various embodiments of the focalization control module 20. Various embodiments of the focalization control module 20 according to the present disclosure includes any number of sub-modules embedded within the focalization control module 20. As can be appreciated, the sub-modules shown in Fig. 2 can be combined and/or further partitioned to similarly generate control signals to the automatic flight system 16, generate control signals to the notification system 17 and/or generate alerts for the user interface 18. Inputs to the focalization control module 20 may be sensed from the aircraft 10 (Fig. 1), received from other control modules (not shown) within the aircraft 10, and/or determined/modeled by other sub-modules (not shown) within the focalization control module 20. In various embodiments, the focalization control module 20 includes a user interface (UI) control module 100, a perseverance control module 102 and a threshold datastore 104.

The threshold datastore 104 stores various expectations or thresholds for determining a focalization state of the pilot. For example, the threshold datastore 104 stores a threshold for an amount of perspiration on a portion of the pilot, a threshold for an eye condition of the pilot, a threshold for a heart rate of the pilot, a threshold for cerebral activity of the pilot, a threshold for pilot input to the flight control input system 32 for a given flight phase and a threshold for pilot input to the switch system 34 for a given flight phase. In various embodiments, one or more of the thresholds are predefined (e.g., factory set). As can be appreciated, the threshold datastore 104 is any non-volatile memory type that stores the information over the repeated operation of the aircraft 10.

The UI control module 100 generates user interface data 110 that may be used by the display 38 to display a user interface that includes an alert for the pilot of the aircraft 10. The UI control module 100 receives as input user input data 112 based on a pilot's interaction with the user input device 36. The user input data 112 comprises a response 114 for the perseverance control module 102. The UI control module 100 also receives alert data 116 from the perseverance control module 102. The alert data 116 comprises a graphical image, a message or combination thereof from which the UI control module 100 generates the user interface data 110 for display on the display 38. In one embodiment, the alert data 116 is generated for a first alert and a second alert, and the first alert is different than the second alert. In addition, in one example, the alert data 116 requests input from the pilot via the user input device 36 and/or flight control system 14 in order to clear the alert. It should be noted that the alert data 116 may also comprise data to remove certain information from the user interface displayed on the display 38, and thus, the alert data 116 described herein is merely exemplary.

The perseverance control module 102 receives as input human parameter data 118, such as perspiration data 120 from the perspiration monitoring system 24, eye condition data 122 from the eye monitoring system 26, heart rate data 124 from the heart rate sensor 28 and cerebral activity data 126 from the cerebral activity monitoring system 30. The perseverance control module 102 also receives as input aircraft controls interaction data 128, such as, flight control input data 130 from the flight control input system 32 and switch input data 132 from the switch system 34. When the human parameter data 118 and aircraft controls interaction data 128 is received, the perseverance control module 102 retrieves from the threshold datastore 104, threshold data 134. Based on a comparison of the human parameter data 118 and aircraft controls interaction data 128 with the threshold data 134, the perseverance control module 102 sets alert data 116 for use by the UI control module 100, outputs one or more control signals 136 for the automatic flight system 16 and outputs one or more control signals 138 for the notification system 17. The one or more control signals 136, when received by the automatic flight system 16, cause the automatic flight system 16 to activate the AFCS. The one or more control signals 138, when received by the notification system 17, cause the control module of the notification system 17 to output a notification to the cockpit. For example, based on the one or more control signals, the notification system 17 activates the haptic device and/or the visual device.

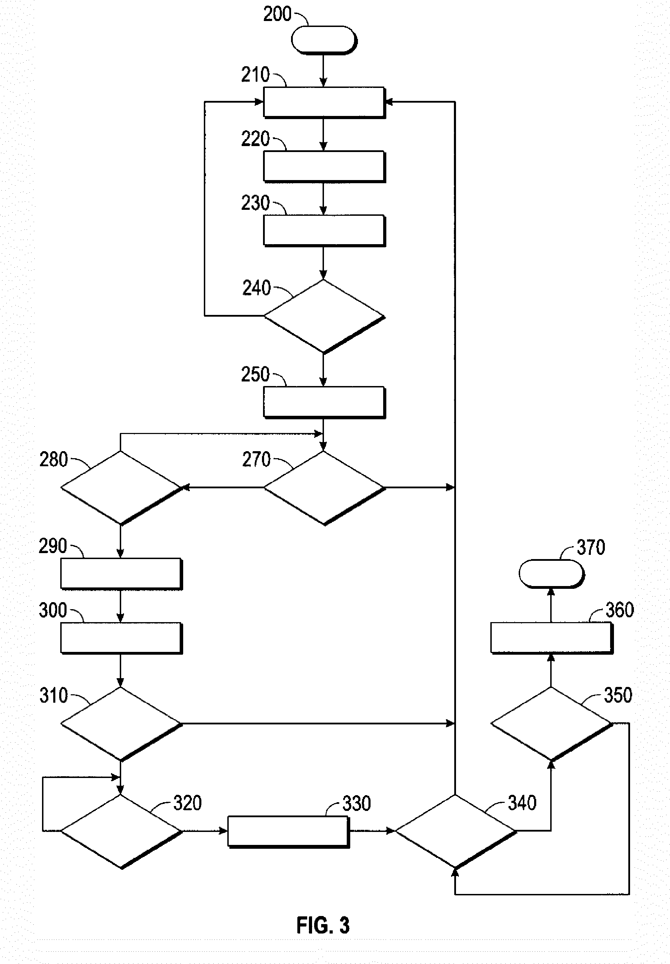

Referring now to Fig. 3, and with continued reference to Figs. 1 and 2, a flowchart illustrates a control method that can be performed by the focalization control module 20 in accordance with the present disclosure. As can be appreciated in light of the disclosure, the order of operation within the method is not limited to the sequential execution as illustrated in Fig. 3, but may be performed in one or more varying orders as applicable and in accordance with the present disclosure. As can further be appreciated, one or more steps of the method may be added or removed without altering the spirit of the method.

The method may begin at 200. Human parameter data 118 is received from one or more of the perspiration monitoring system 24, the eye monitoring system 26, the heart rate sensor 28 and the cerebral activity monitoring system 30 at 210. Aircraft controls interaction data 128 is received from one or more of the flight control input system 32 and switch system 34 at 220. At 230, the threshold datastore 104 is queried for threshold data 134 associated with the human parameter data 118 and aircraft controls interaction data 128. At 240, the method determines if the human parameter data 118 or the aircraft controls interaction data 128 exceeds a threshold from the threshold data 134.

For example, if the human parameter data 118 includes perspiration data 120, the method determines if the measured perspiration is greater than a threshold perspiration amount. In another example, if the human parameter data 118 includes eye condition data 122, the method determines at least one of if an amount of time spent by the pilot looking at an object is greater than a threshold amount of time, such as about 20 seconds, if a switching rate of the eyes of the pilot are greater than a threshold eye switching rate, if a pupil dilation of an eye of the pilot is greater than a threshold pupil dilation amount, and if a fixation/saccades ratio for the pilot's eyes is greater than a threshold fixation/saccades ratio during a predefined period of time, such as about 20 seconds. As a further example, if the human parameter data 118 includes heart rate data 124, the method determines if the measured heart rate is greater than a threshold heart rate, such as about 110 beats per minute (bpm). In another example, if the human parameter data 118 includes cerebral activity data 126, the method determines at least one of if the cerebral activity in the theta band in the medial frontal regions is greater than a threshold range for cerebral activity in the theta band, such as about 5-7 Hertz (Hz), if the cerebral activity in the alpha band in the medial frontal regions is greater than a threshold range for cerebral activity in the alpha band, such as 8-12 Hz, for an EEG-EP if the intensity measured is less than a threshold intensity, and for an SPIf, if a level of blood oxygenation in a prefrontal region is greater than a threshold for blood oxygenation in the prefrontal region during a predefined period of time, such as about 20 seconds.

With regard to the aircraft controls interaction data 128, in one example, the method determines if the flight control input data 130 exceeds a threshold for flight control input for a given flight phase (e.g. take-off, cruising, landing). For example, the method determines if pilot input to the flight control input system 32 exceeds a threshold amount for a given flight phase, including, but not limited to, if a pilot input to the stick is greater than a threshold stick input for the flight phase, if a pilot input to the rudder control is greater than a threshold input for the flight phase or if pilot input to the throttle is greater than a threshold input for the flight phase. The method also determines, if the switch input data 132 exceeds a threshold for the flight phase. For example, the method determines if the input received to a particular switch of the switch system 34 is acceptable or within threshold limits for the given flight phase. In addition, the method may also determine if user input data 112 received by the UI control module 100 exceeds a threshold for the current flight phase.

If at 240, the method determines that the human parameter data 118 or the aircraft controls interaction data 128 exceeds the threshold from the threshold data 134, the method goes to 250. Otherwise, the method loops to 210. At 250, a timer T₁ is activated. At 270, the method determines if a response 114 has been received from the user input data 112. If a response 114 has been received, then the method goes to 210.

Otherwise, at 280, the method determines if the time of the timer T₁ exceeds a predetermined period of time, such as about 20 seconds. If the timer T₁ exceeds the predetermined period of time, at 290, the method starts a timer T₂. At 300, alert data 116 is generated for the UI control module 100 and/or one or more control signals 138 are generated for the notification system 17 to provide a first alert. In one example, for the first alert, the first alert is displayed on the display 38 via the user interface data 110 or the visual device of the notification system 17 is activated via the receipt of the one or more control signals 138.

At 310, the method determines if input has been received from the flight control input data 130 or switch input data 132. If input has been received, the method goes to 210. Otherwise, at 320, the method determines if the timer T₂ exceeds a predetermined period of time, such as about 15 seconds.

If the timer T₂ exceeds the predetermined period of time, at 330, alert data 116 is generated for the UI control module 100 and/or the one or more control signals 138 are generated for the notification system 17 to provide a second alert. Generally, the alert data 116 for the second alert requires the pilot to provide input to the flight control system 14 and/or the switch system 34 in order to clear the second alert. In addition, the alert data 116 associated with a second alert may comprise alert data 116 to the UI control module 100 remove a portion of the data displayed in the user interface data 110. For example, based on the human parameter data 118, such as eye condition data 122, the focalization control module 20 sets alert data 116 for the second alert that includes a graphical and/or textual flight control command to the pilot along with data to remove a portion of the user interface to which the pilot is focused based on the eye condition data 122. It should be noted that the first alert and the second alert described herein are merely exemplary, as any escalating alerting scheme could be employed to redirect the focus of the pilot.

At 340, the method determines, based on the flight control input data 130 or switch input data 132, if an input has been received to the flight control system 14. If an input has been received, then the method loops to 210. Otherwise, at 350, the method determines if a predetermined time period has been exceeded for receipt of the input to the flight control system 14, such as, for example about 15 seconds. If the predetermined time period has not been exceeded, the method loops. Otherwise, at 360, the one or more control signals 136 are generated for the automatic flight system 16. Upon receipt of the one or more control signals 136, the automatic flight system 16 activates the AFCS. The method ends at 370. It should be noted that 360 is optional, and the method may end after the expiration of the predetermined time period at 350.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the disclosure in any way. Rather, the foregoing detailed description will provide those skilled in the art with a convenient road map for implementing the exemplary embodiment or exemplary embodiments. It should be understood that various changes can be made in the function and arrangement of elements without departing from the scope of the appended claims and the legal equivalents thereof.

## Claims

1. Method of determining focalization of a pilot of an aircraft (10), **characterized in that** it comprises the following steps:
receiving human parameter data (120,122,124,126) from one or more systems (24,26,28,30) that observe conditions associated with the pilot;
receiving controls data (128) that indicates an interaction of the pilot with one or more control systems (14) of the aircraft;
based on the human parameter data and the controls data, generating at least one alert; and
communicating the at least one alert to the pilot to change a focus of the pilot.

2. Method according to claim 1, wherein the at least one alert comprises a first alert and a second alert, and the method further comprises:
displaying the first alert on a display (38) of a user interface (18).

3. Method according to claim 2, further comprising:
receiving a response from a user input device (36) associated with the user interface based on the first alert being displayed on the user interface (18).

4. Method according to claim 3, further comprising:
generating the second alert if the response is not received before the end of a first predetermined time period.

5. Method according to claim 4, further comprising:
displaying the second alert on the display of the user interface (18), wherein the second alert is different than the first alert.

6. Method according to claim 5, further comprising:
outputting one or more control signals (136) to an automatic flight system (16) of the aircraft if user input to the one or more flight control systems (14) of the aircraft is not received before the end of a second predetermined time period.

7. Method according to claim 4, wherein generating the second alert further comprises:
generating one or more control signals (138) to a notification system associated with the aircraft (10) to activate at least one of a visual device or a haptic device located within a cockpit of the aircraft.

8. Method according to any one of the preceding claims, wherein receiving human parameter data further comprises:
receiving at least one of perspiration data, eye condition data, heart rate data and cerebral activity data.

9. Method according to claim 1, wherein generating the at least one alert further comprises:
providing a datastore (104) that stores threshold data for the human parameter data (120,122,124,126) and the controls data;
comparing the human parameter data and the controls data to the threshold data; and
generating the at least one alert if at least one of the human parameter data and the controls data exceeds a threshold of the threshold data.

10. An aircraft (10) comprising:
at least one human monitoring system (12) that observes a condition associated with a pilot of the aircraft and generates human parameter data (120,122,124,126) based on the observed condition;
at least one flight control system (14) that receives pilot input for controlling the operation of the aircraft (10);
a user interface (18) including a user input device (36) for receiving user input data from the pilot and a display (38);
**characterized in that** it comprises a focalization control module (20) that generates a first alert based on the human parameter data or the pilot input to the at least one flight control system (14), the first alert output for display on the display, and the focalization control module generates a second alert based on the user input data received from the pilot via the user input device.

11. An aircraft according to claim 10, wherein the second alert is generated upon the expiration of a first predetermined time period if user input is not received by the user input device (36).

12. An aircraft according to claim 11, wherein the second alert is output to the display, and if pilot input is not received by the at least one flight control system before the expiration of a second predetermined time period, the focalization control module (20) generates one or more control signals for an automatic flight system (16) of the aircraft.

13. An aircraft according to claim 10, wherein the at least one human monitoring system includes a perspiration monitoring system (24) that observes a perspiration condition on a portion of the pilot and generates perspiration data based on the observed condition.

14. An aircraft according to claim 10, wherein the at least one human monitoring system includes an eye monitoring system (26) that observes conditions of the eyes of the pilot and generates eye condition data based on the observed conditions.

15. An aircraft according to claim 14, wherein the focalization control module (20) generates the first alert based on the human parameter data if the eye condition data exceeds a threshold.

16. An aircraft according to claim 10, wherein the focalization control module (20) generates the first alert based on the pilot input if the pilot input for a flight phase of the aircraft exceeds a threshold for the flight phase.
